# EUROPEAN PATENT SPECIFICATION

(11) **EP 0 515 755 B1**
(45) Date of publication and mention of the grant of the patent: **01.03.1995**
(21) Application number: 91401383.4
(22) Date of filing: 29.05.1991
(51) Int. Cl.: A23L 1/29, A61K 35/78

(54) **A diet for suppressing the absorption of saccharides**
Diät zur Unterdrückung der Absorption von Sacchariden
Diète pour la suppression de l'absorption de saccharides

(43) Date of publication of application: 02.12.1992
(73) Proprietor: KOWA CHEMICAL INDUSTRIES CO., LTD., Toyonaka-shi, Osaka (JP)
(72) Inventor: Kazuo, Iwasaki, Ota-ku, Tokyo (JP); Chiaki, Yamashita, Mitaka-shi, Tokyo (JP); Shahram, Mesri, Tokyo (JP); Yoshio, Iwasaki, Tokyo (JP)
(74) Representative: Kedinger, Jean-Paul

(56) References cited:
- PATENT ABSTRACTS OF JAPAN vol. 13, no. 101 (C-574)(3449), 9 March 1989; & JP - A - 63277627 (NITTO ELECTRIC IND. CO.LTD.) 15.11.1988
- PATENT ABSTRACTS OF JAPAN vol. 13, no. 277 (C-600)(3575), 25 May 1989; & JP - A - 138026 (NITTO ELECTRIC IND. CO. LTD.)08.02.1989
- PATENT ABSTRACTS OF JAPAN vol. 10, no. 144 (C-
- 349)(2201), 27 May 1986; & JP - A - 615023 (YASUTAKE HICHI) 10.01.1986
- PATENT ABSTRACTS OF JAPAN vol. 12, no. 496 (C-555)(3343), 23 December 1988;&& JP - A - 63208532 (YASUTAKE NITSUCHI) 30.08.1988

## Description

### BACKGROUND OF THE INVENTION

The present invention relates to a diet for suppressing the absorption of saccharides from the intestines.

As it is called an age of satiation, in recent years, people have come to enjoy the table full of variety and delicacies, and while the intake of sugars, starches, and the like becomes excessive, the quantity of people's exercise has decreased, so that people have become corpulent from their early childhood and are susceptible to various geriatric diseases such as diabetes mellitus.

As means of preventing one from having corpulence that will induce geriatric diseases, diet therapies wherein the intake of starches and the like is restricted are followed, but these therapies are utterly weary and require one's strong determination. Therefore, artificial sweetenings that are not absorbed into the intestines are developed and used, but these only decrease the amount of sugars and have no relation with the absorption of saccharides originated from carbohydrates, so that they cannot be sweeping measures.

To cure a diabetic, a method wherein insulin is administered to suppress the increase in blood sugar or a method wherein various pharmaceutics are administered to accelerate the secretion of insulin from cells or Langerhans's islands of the pancreas is taken but any of them is accompanied by diet restriction and the present state is such that the diabetic is forced to have painful and dull food life for a long period of time.

Therefore, diets that comprise, as raw material, Gymnema sylvestre produced in India having an effect for suppressing the absorption of saccharides and are intended for the prevention of corpulence and for the suppression of the increase of blood sugar have been suggested, for example, in Japanese Patent Application Laid-Open Nos. 5023/1986 and 208532/1988.

However since the leaf and extract of Gymnema sylvestre are strong in bitterness and astringency and act on the sweetness receptor of taste cells in the mouth thereby exhibiting a sweetness sensation suppressing effect that prevents the combination of a sweetening with the sweetness receptor, the commercialization using it requires various measures.

For instance, Gymnema sylvestre can be used as an oral food only by reducing or preventing the bitterness, the astringency, or the sweetness sensation suppressing effect through many troublesome processes, for example, by causing an enzyme to act on the Gymnema sylvestre in the presence of a farinaceous substance to reduce the bitterness (Japanese Patent Application Laid-Open Nos. 2552/1989 and 102028/1989), by adding a cyclodextrin to the extract thereby enclosing it (Japanese Patent Application Laid-Open No. 120263/1989), by adding a fat-soluble compound (Japanese Patent Application Laid-Open No. 27327/1988), or by coating with a polymeric substance (Japanese Patent Application Laid-Open No. 38026/1989).

### SUMMARY OF THE INVENTION

The object of the present invention is to provide a diet for suppressing the absorption of saccharides that is free from unpleasant tastes such as bitterness and astringency, has barely a sweetness sensation suppressing effect, suppresses the absorption of saccharides through the intestines, and can be prepared without requiring any troublesome process.

The inventors have continued researches to solve the above problems, have tested and studied Gymnema inodrum, a liana, that grows naturally in the tropics, for example, in Malaysia and Thailand, and have found that the extracted component therefrom is excellent in action for suppressing the absorption of saccharides.

That is, the present invention is directed to a diet for suppressing the absorption of saccharides whose raw material is Gymnema inodrum and which is, for example, in a liquid form like tea prepared by drying and roasting the leaves of Gymnema inodrum or in the form of tablets or granules prepared by subjecting the leaves of Gymnema inodrum to extraction with hot water, alcohol or the like, and condensing and drying the extracted component, which tablets or granules will be taken as such or will be added to a food or a drink such as a drinking agent and confectionery.

Since the present diet for suppressing the absorption of saccharides uses, as a raw material, Gymnema inodrum having an effect for suppressing the absorption of saccharides, if one takes it in the same amount of saccharides as conventional, the absorption can be small and one can reduce calories without unnaturally restricting his diet.

Further, since Gymnema inodrum, in comparison with Gymnema sylvestre having the same effect for suppressing the absorption of saccharides, has no unpleasant tastes such as bitterness and astringency and has almost no sweetness suppressing effect, it does not require troublesome processing such as mixing other components to mitigate bitterness or coating with other substance.

### BRIEF DESCRIPTION OF THE DRAWINGS

Fig. 1 is a diagram showing an effect of Gymnema inodrum extraction on high K⁺-induced contraction of the ileal longitudinal muscle isolated from guinea pig;
Fig. 2 is a diagram showing a glucose removal on high K⁺-induced contraction of the ileal longitudinal muscle isolated from guinea pig; and
Fig. 3 is a diagram showing a suppressive effect of GI extraction on the blood sugar level with time.

### DETAILED DESCRIPTION

After 100 g of dried leaves of Gymnema inodrum was soaked and stirred in 1 liter of hot water at 80 °C for 8 hours, the liquid was condensed in a thin-layer evaporator to about 250 ml, so that an extract (GI solution) having a solid content of 5.6 g/100 ml was obtained.

### 1) Assay of the saccharide absorption suppressing effect

The action of the extracted component of Gymnema inodrum on the high K⁺-induced contraction of the ileal longitudinal muscle of a guinea pig was examined by the Magnus method.

That is, the response of the ileal muscle strip suspended in a Magnus bath at 37 °C to the above extract component was magnified by a transducer and an amplifier and was drawn in a recorder.

Tyrode solution (a modified physiological salt solution: PSS) was bubbling with gas mixture of 95 % O₂ + 5 % CO₂ keeping at pH 7.2.

First, the ileal muscle was suspended in 15 ml of PSS, and after one hour of equilibration, PSS solution was replaced with a high K⁺ Tyrode solution prepared by adding hyperosmotically 60 mM of potassium chloride (KCl) to PSS so as to contract the muscles as reference.

After the incubation of about 30 minutes, the high K⁺ solution was replaced with PSS to relax the muscle. After twice application of the high K⁺ solution to the muscle, by adding 0.75 ml of a diluted solution of the GI solution under the conditions wherein the muscle is contracted by the high K⁺ solution and after 120 min, replaced with 11mM pyruvate solution, the change of the tension was recorded.

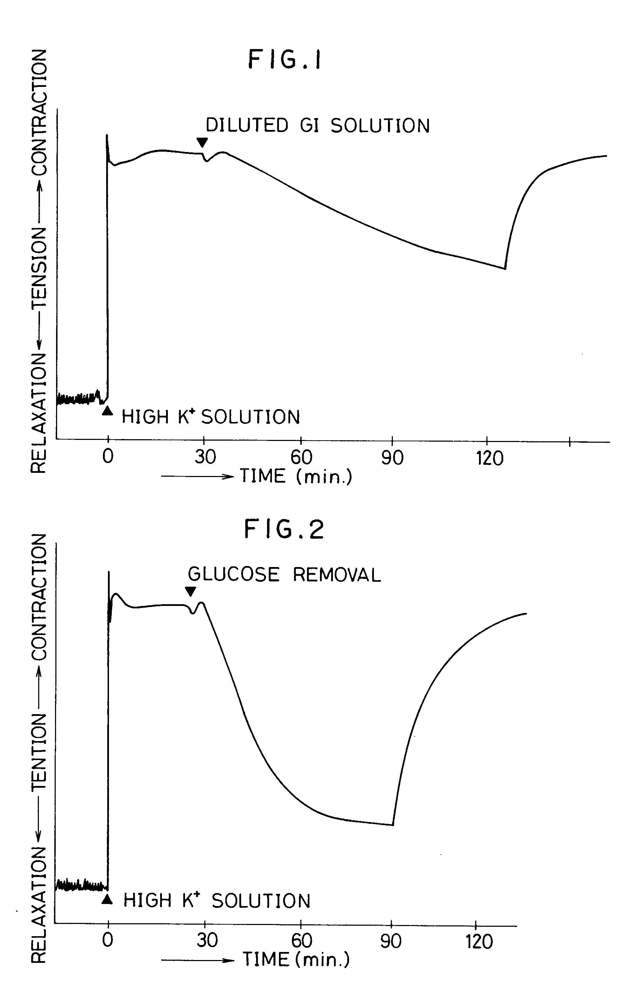

A typical tension change curve of the muscle which 0.75 ml of a 20-time dilute solution of the GI solution is shown in Fig. 1.

The developed tension by the high K⁺ solution was decreased.when the high K⁺ Tyrode solution was replaced with a glucose-depleted high K⁺ solution, and after 90 min. replaced with 11mM pyruvate solution is shown in Fig. 2.

As shown in Fig. 1, the muscle relaxed by adding the GI solution. That is, it is assumed that the uptake and/or utilization of glucose is suppressed.

### 2) Assay of suppressing effect on the blood sugar rise

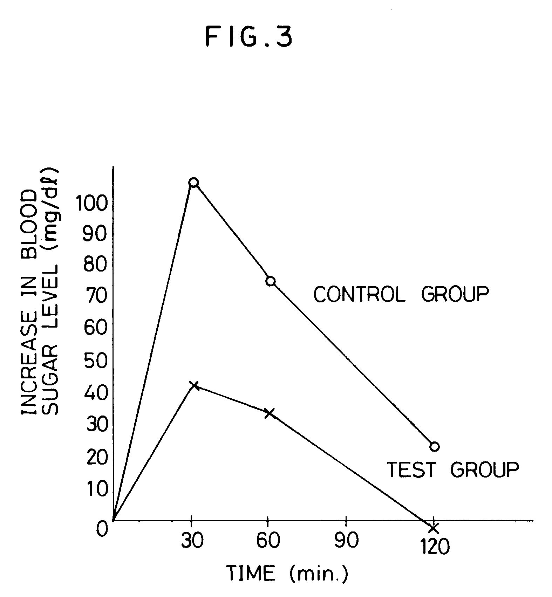

The rise of sugar blood level from the time of the evacuation of the intestines was checked by the oral glucose tolerance test using Wistar-Imamichi-rats (weight: 200 to 300 g) to which was administered dried powder of an extract obtained by freeze-drying the above GI solution. That is, the rats for the test were fasted one night before the test. With respect to a control group of the rats to which only glucose was administered in an amount of 1 g/kg of body weight and a test group of the rats to which glucose in an amount of 1 g/kg of body weight and the above extract component in an amount of 0.1 g/kg of body weight, the blood sugar level before the administration and 30 minutes, 60 minutes, and 120 minutes after the administration determined. The results are shown in Fig. 3. The blood sugar level at the time of the evacuation of the intestines was 96 + 3 mg/dl.

As indicated in Fig. 3, it is suggested that the extract component of Gymnema inodrum suppresses the absorption of glucose and also the rise of blood sugar level.

### 3) Comparison with Gymnema sylvestre

500 g of each of Gymnema sylvestre produced in India and Gymnema inodrum produced in Thailand which were prepared by washing the raw leaves with water and drying in the sun was placed in a roaster and was roasted at 200 °C for 15 minutes while rolling the leaves, and then they were immediately taken out and were allowed to stand to cool to room temperature.

200 ml of hot water was poured to 1 g of each of the two kinds of the roasted leaves, and after they were allowed to stand for 3 minutes, they were filtered through a wire mesh and were subjected to the following organoleptic test.

Test items are odor, bitterness, and sweetness, which were assessed by 10 panelists on a 1-5 scale.

Odor: the peculiar odor when the tea liquid was put in the mouth.

Bitterness: the aftertaste when the tea liquid was swallowed.

Change of sweetness: A commercially available Yokan (sweet jelly of beans) was tasted before and after the drinking of the tea liquid, and the change of the degree of developed bitterness was assessed.

**Table 1**

| | Odor | Bitterness | Change of sweetness |
|---|---|---|---|
| Gymnema inodrum | 1 | 1 | 1 |
| Gymnema sylvestre (control) | 5 | 5 | 5 |

As it is clear in Table 1, in comparison with the tea liquid of Gymnema sylvestre, that of Gymnema inodrum is almost free from peculiarity, so that it is very advantageous to use the Gymnema inodrum in various uses.

## Claims

1. A diet for suppressing the absorption of saccharides which uses Gymnema inodrum as a raw material.

2. A diet for suppressing the absorption of saccharides as claimed in claim 1, which is prepared by drying and then roasting the leaves of Gymnema inodrum.

3. A diet for suppressing the absorption of saccharides as claimed in claim 1, which is prepared by subjecting the leaves of Gymnema inodrum to extraction with hot water and condensing the extract.

4. A diet for suppressing the absorption of saccharides as claimed in claim 1, which is prepared by subjecting the leaves of Gymnema inodrum extraction with an alcohol and condensing the extract.

5. A diet for suppressing the absorption of saccharides as claimed in claim 1, which is prepared by subjecting the leaves of Gymnema inodrum extraction with hot water and drying the extract.

6. A diet for suppressing the absorption of saccharides as claimed in claim 1, which is prepared by subjecting the leaves of Gymnema inodrum extraction with an alcohol and drying the extract.

## Patentansprüche

1. Diät zur Unterdrückung der Aufnahme von Sacchariden, unter Anwendung von Gymnema Inodrum als Rohstoff.

2. Diät zur Unterdrückung der Aufnahme von Sacchariden nach Anspruch 1, die durch Trocknen und anschließendes Rösten der Gymnema Inodrum Blätter vorbereitet wird.

3. Diät zur Unterdrückung der Aufnahme von Sacchariden nach Anspruch 1, die durch Ausziehen der Gymnema Inodrum Blätter mit heißem Wasser und Kondensieren des Auszugs vorbereitet wird.

4. Diät zur Unterdrückung der Aufnahme von Sacchariden nach Anspruch 1, die durch Ausziehen der Gymnema Inodrum Blätter mit Alkohol und Kondensieren des Auszugs vorbereitet wird.

5. Diät zur Unterdrückung der Aufnahme von Sacchariden nach Anspruch 1, die durch Ausziehen der Gymnema Inodrum Blätter mit heißem Wasser und Trocknen des Auszugs vorbereitet wird.

6. Diät zur Unterdrückung der Aufnahme von Sacchariden nach Anspruch 1, die durch Ausziehen der Gymnema Inodrum Blätter mit Alkohol und Trocknen des Auszugs vorbereitet wird.

## Revendications

1. Produit de régime pour supprimer l'absorption de saccharides, utilisant comme matière de base le Gymnema inodrum.

2. Produit de régime pour supprimer l'absorption de saccharides, tel que défini dans la revendication 1, préparé par séchage et puis grillage des feuilles de Gymnema inodrum.

3. Produit de régime pour supprimer l'absorption de saccharides, tel que défini dans la revendication 1, préparé en soumettant les feuilles de Gymnema inodrum à une extraction avec de l'eau chaude et en concentrant l'extrait.

4. Produit de régime pour supprimer l'absorption de saccharides, tel que défini dans la revendication 1, préparé en soumettant les feuilles de Gymnema inodrum à une extraction avec un alcool et en concentrant l'extrait.

5. Produit de régime pour supprimer l'absorption de saccharides, tel que défini dans la revendication 1, préparé en soumettant les feuilles de Gymnema inodrum à une extraction avec de l'eau chaude et en déshydratant l'extrait.

6. Produit de régime pour supprimer l'absorption de saccharides, tel que défini dans la revendication 1, préparé en soumettant les feuilles de Gymnema inodrum à une extraction avec un alcool et en déshydratant l'extrait.
